(19) Europäisches Patentamt
European Patent Office
Office européen des brevets

(11) Numéro de publication : **0 432 021 A1**

(12) **DEMANDE DE BREVET EUROPEEN**

(21) Numéro de dépôt : **90403395.8**

(22) Date de dépôt : **29.11.90**

(51) Int. Cl.$^5$ : **C07D 317/20**, C07D 317/16, C07F 9/655, C07C 403/14, C07C 317/10

(30) Priorité : **01.12.89 FR 8915870**

(43) Date de publication de la demande : **12.06.91 Bulletin 91/24**

(84) Etats contractants désignés : **AT BE CH DE DK ES FR GB GR IT LI LU NL SE**

(71) Demandeur : **RHONE-POULENC NUTRITION ANIMALE**
**Rue Marcel Lingot**
**F-03600 Commentry (FR)**

(72) Inventeur : **Chabardes, Pierre**
**24 rue Jeanne d'Arc**
**F-69110 Sainte Foy Les Lyon (FR)**
Inventeur : **Duhamel, Lucette**
**32 rue Jacques Boutrolles**
**F-76130 Mont Saint-Aignan (FR)**
Inventeur : **Duhamel, Pierre**
**32 rue Jacques Boutrolles**
**F-76130 Mont Saint-Aignan (FR)**
Inventeur : **Guillemont, Jérôme**
**7 rue Rioult**
**F-27210 Beuzeville (FR)**
Inventeur : **Poirier, Jean-Marie**
**17 Impasse de la Grande Madeleine**
**F-76160 Saint-Martin du Vivier (FR)**

(74) Mandataire : **Le Pennec, Magali et al**
**RHONE-POULENC RORER SA, Direction des Brevets, 20 Avenue Raymond Aron**
**F-92165 Antony Cédex (FR)**

(54) **Nouveaux intermédiaires, leur procédé de préparation et leur utilisation pour la synthèse des vitamines A et E.**

(57) Nouveaux intermédiaires de préparation des vitamines A et E qui sont les acétals du dihalogéno-5,6 méthyl-3 hydroxy-3 hexanal ou de l'halogéno-6 hydroxy-3 méthyl-3 hexène-5 al ou du méthyl-3 hydroxy-3 hexène-5 al.

Ces intermédiaires après deshydrohalogénation et deshydratation sont condensés directement sur la β-ionone ou sur la méthyl-2 heptène-2 one-6 pour synthétiser les vitamines A et E respectivement.

**EP 0 432 021 A1**

# NOUVEAUX INTERMEDIAIRES, LEUR PROCEDE DE PREPARATION ET LEUR UTILISATION POUR LA SYNTHESE DES VITAMINES A ET E

La présente invention concerne de nouveaux intermédiaires de préparation de vitamines, leur procédé de préparation et leur utilisation pour la synthèse des vitamines A et E. Ces nouveaux intermédiaires contiennent 7 atomes de carbone et peuvent être directement condensés sur la β-ionone ou sur des sulfones contenant 13 atomes de carbone pour la préparation de la vitamine A.

Parmi les procédés de préparation de la vitamine A condensant directement un dérivé contenant 7 atomes de carbone on peut citer le brevet EP242247 gui décrit la condensation d'un bromo-6 méthyl-3 trialkylsilyloxy-1 hexatriène avec la β-ionone. Ce type de condensation est particulièrement performant, le seul inconvénient est le coût prohibitif de la matière première silylée. En effet, la préparation du dérivé silylé à partir du diméthoxy-5,5 méthyl-3 pentène-2 al exige trois étapes dont certaines très longues, l'utilisation de matières premières telles que le bromure de bromométhyltriphénylphosphonium ; ce procédé ne permet d'obtenir que des rendements globaux ne dépassant pas 30% en dérivé silylé à partir d'un dérivé comportant 6 atomes de carbone.

Il existe également une voie différente partant du dérivé lithié du composé contenant 7 atomes de carbone gui consiste à le mettre en présence de la β-ionone. La matière première contenant 7 atomes de carbone est le bromo-6 méthyl-3 diméthoxy-1,1 hexadiène-3,5. Ce dernier composé est préparé à partir de bromométhyltriphénylphosphonium préparé selon l'article paru dans Tétrahedron Letters (Matsumoto, Kurode 1980, 21, 4021) et du diméthoxy-1,1 méthyl-3 pentène-2 al gui lui-même est préparé selon le brevet GB 1591968 à partir d'orthoformiate d'alcool et d'énoxysilane contenant une chaîne alkényle contenant 4 atomes de carbone. Cette méthode d'accès aux dérivés contenant 7 atomes de carbone est particulièrement longue et coûteuse.

L'industrie cherche depuis longtemps un procédé simple à partir de matières premières peu onéreuses de préparation de la vitamine A.

La présente invention a permis d'atteindre cet objectif.

Elle permet de synthétiser la vitamine A ou la vitamine E à partir de matière première peu chère et disponible telle que l'acétal diméthylique de l'acétylacétaldéhyde.

La présente invention concerne de nouveaux motifs contenant 7 atomes de carbone et répondent à la formule générale (I) suivante :

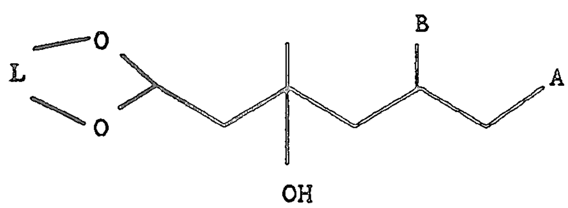

(I)

dans laquelle

- L représente un radical alkylène contenant 2 à 3 atomes de carbone éventuellement substitué par un ou plusieurs groupes alkyle, alkylène ou alkoxy contenant 1 à 4 atomes de carbone,
- A représente un halogène choisi parmi le chlore, le brome et l'iode et/ou une liaison covalente avec B,
- B représente un halogène choisi parmi le chlore, le brome et l'iode ou une liaison covalente avec A,

On préfère parmi les nouveaux motifs contenant 7 atomes de carbone ceux pour lesquels L représente un groupe alkylène et l'halogène est le brome.

Parmi ces composés on préfère tout particulièrement :

- l'éthylènedioxy-1,1 hydroxy-3 méthyl-3 hexène-5
- le dibromo-5,6 éthylènedioxy-1,1 méthyl-3 hexanol-3
- le bromo-6 éthylènedioxy-1,1 hydroxy-3 méthyl-3 hexène-5

Le procédé de préparation des composés de formule (I) dans laquelle A représente une liaison covalente avec B consiste dans une première étape à mettre en présence l'acétal dialkylique de l'acétylacétaldéhyde représenté par la formule (A) :

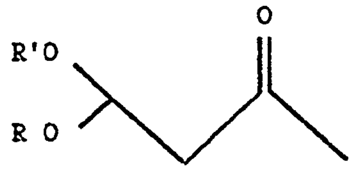

(A)

dans laquelle R et R′ représentent chacun un groupe alkyle linéaire ou ramifié contenant 1 à 4 atomes de carbone identique ou différent avec un dialcool en présence d'un catalyseur acide.

Le dialcool est de préference un dialcool aliphatique contenant 2 à 3 atomes de carbone éventuellement substitué par un plusieurs groupes alkyle alkylène ou alkoxy contenant 1 à 4 atomes de carbone. On préfère tout particulièrement utiliser l'éthylène glycol ou le propylène glycol ainsi que les dérivés de l'éthane diol de formule :

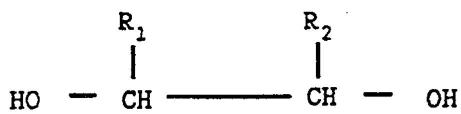 ou du

propane diol de formule :

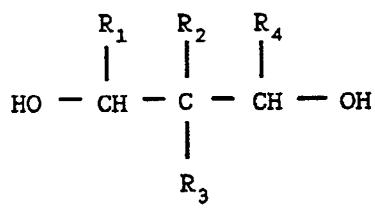

dans lesquelles $R_1$, $R_2$, $R_3$, $R_4$ représentent des groupes identiques ou différents choisis parmi les radicaux alkyle, alkylène ou alkoxy contenant 1 à 4 atomes de carbone. Il est bien évident que deux groupes R alkylène peuvent former un cycle alicyclique.

On préfère tout particulièrement utiliser l'éthylène glycol. Le catalyseur acide est choisi notamment parmi les acides de Lewis (chlorure de zinc, d'aluminium, trifluorure de bore) l'acide paratoluène sulfonique.

La deuxième étape du procédé de préparation consiste à mettre en contact le produit obtenu à la première étape qui est de préference l'éthylènedioxy-1,1 butanone-3 avec un halogènure d'allyl magnésium de préférence le chlorure.

La réaction est réalisée de préférence dans un solvant inerte dans les conditions de la réaction tel que les éthers à une température comprise entre -20°C et 0°C.

Le procédé de préparation des composés de formule (I) dans laquelle A et B représentent un halogène, consiste à mettre en contact l'alkylènedioxy-1,1 hydroxy-3 méthyl-3 hexène-5 avec l'halogène moléculaire. Cette réaction est réalisée de préférence dans un solvant inerte dans les conditions de réaction tel que les solvants halogénés aliphatiques (tétrachlorure de carbone) ou aromatiques (chlorobenzènes) à une température comprise entre -20°C et 10°C en présence d'une base faible telle que les carbonates alcalins.

Le procédé de préparation des composés de formule (I) dans laquelle A représente un halogène, B représente une liaison covalente avec A, consiste à traiter le produit brut précédent par une base forte telle que le tertiobutylate de potassium.

Les dérivés de formule (I) dans lesquels A et B représentent chacun un halogène sont utilisés pour la préparation du rétinal ou de la vitamine E par un procédé qui consiste à éliminer un halogène et le groupe hydroxyle au moyen d'une base choisie de préférence parmi les amines tertiaires, les alcoolates et d'un halogénure tel que le chlorure de méthane sulfonyle ou l'oxychlorure de phosphore ($POCl_3$).

Le dérivé obtenu, l'halogéno-6 alkylènedioxy-1,1 méthyl-3 hexadiène-3,5, peut être condensé après transmétallation au moyen d'un alkyl lithium sur la β-ionone gui après déhydroxylation et déprotection au moyen d'un acide fort dans un solvant organique de préférence l'acétone donne le rétinal. L'halogéno-6 alkylènedioxy-1,1 méthyl-3 hexadiène-3,5 donne également après condensation sur la méthyl-2 heptène-2 one-6 et dehydratation le dehydrofarnesal.

Les composés de formule (I) dans lesquels A représente un halogène et B une liaison covalente avec A sont eux aussi utilisés pour la synthèse de la vitamine A ou E par un procédé analogue à celui décrit précédemment qui consiste à effectuer une déhydratation au moyen d'une base choisie de préférence parmi les amines tertiaires, les alcoolates et d'un halogénure tel que le chlorure de méthane sulfonyle ou l'oxychlorure de phosphore. Ils conduisent de la même façon à l'halogéno-6 alkylène dioxy-1,1 méthyl-3 hexadiène-3,5.

Le dérivé de formule (I) pour lesquels A et B représentent un halogène peuvent être déprotégés et deshydroxylés par utilisation d'un acide fort dans un solvant organique de préférence l'acétone. On obtient alors le bromo-6 méthyl-3 hexadiène-2,4 al qui après protection au moyen d'un dialcool aliphatique contenant 2 à 3 atomes de carbone ou d'un orthoformiate mixte obtenu par condensation d'un orthoformiate et d'un glycol donne un produit nouveau qui est l'halogéno-6 alkylènedioxy-1,1 méthyl-3 hexadiène-2,4 représenté par la formule générale (II) :

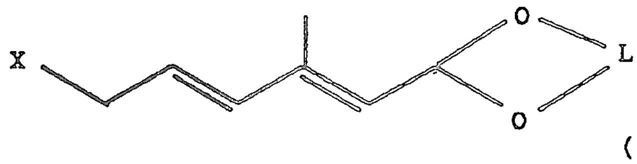

(II)

dans laquelle X et L ont la même signification que dans la formule (I).

Ce composé de formule (II) est condensé sur une sulfone de formule :

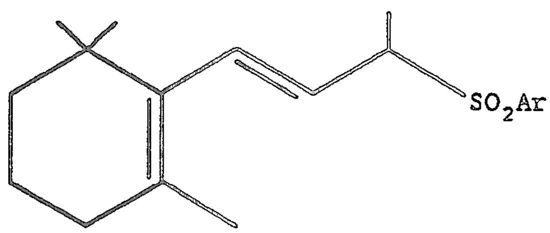

dans laquelle Ar représente un radical aromatique en présence d'un agent basique choisi parmi les hydroxydes, les alcoolates et les hydrures ou amidures alcalins pour donner, après hydrolyse, le rétinal.

Le dérivé de formule (II) par condensation avec un phosphite d'alkyle donne un intermédiaire nouveau de formule (III) :

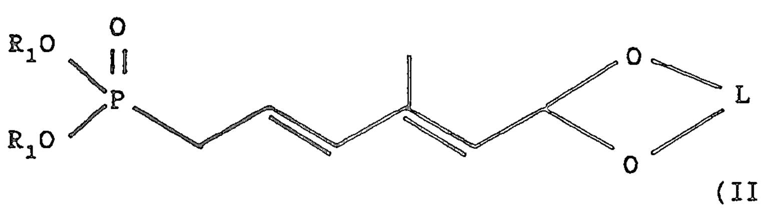

(III)

dans laquelle $R_1$ représente un groupe alkyle linéaire ou ramifié contenant 1 à 6 atomes de carbone, de préférence un groupe éthyle.

Ce composé de formule (III) est condensé directement avec la β-ionone en présence d'une base dans un solvant pour conduire au dioxolanne du rétinal ; celui-ci est traité par un acide fort dans un solvant pour obtenir le rétinal. Il peut de même, à partir de la méthyl-2 heptène-2 one-6 donner le déhydrofarnésal qui est un intermédiaire important dans la synthèse de la vitamine E.

La présente invention sera plus complètement décrite à l'aide des exemples suivants qui ne doivent pas être considérés comme limitatifs de l'invention.

EXEMPLE

I HYDROXYACETALS EN "C7" REPONDANT A LA FORMULE (I)

Elaboration du squelette de base en "C7" :

Dans un claisen de 100 ml, 0,5 mol d'éthylène glycol (31 g) fraîchement distillé et 0,55 mol de diméthoxy-1,1 butanone-3 (72,6 g) sont chauffés à 65°C en présence d'une quantité catalytique d'acide paratoluène sulfonique (100 mg). On recueille le méthanol libéré au cours de la transacétalisation. Une fois le volume théorique obtenu (32 ml), le cétoacétal est distillé sous pression réduite. On prépare ainsi avec un rendement de 80 %:

Ethylènedioxy-1,1butanone-3 (A)

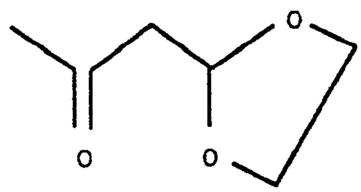

Dans un réacteur sec d'un litre, équipé d'une agitation mécanique, d'un thermomètre, d'un réfrigérant à boule et d'une ampoule à brome, on introduit 0,6 mol de tournure de magnésium (14,6 g) et 100 ml d'éther anhydre. On additionne tout d'abord l'halogénure pur (3 g de chlorure d'allyle). La formation du magnésien est caractérisée par l'apparition d'un trouble dans la solution et d'une augmentation de la température. Le reste du dérivé halogéné (29,1 g, soit au total 32,1 g (0,42 mol)) dilué dans 200 ml d'éther anhydre est ajouté en 60 mn à 0°C. Pour éviter la prise en masse du magnésien, on introduit 200 ml d'éther (par portion de 50 ml) au cours de l'addition du chlorure d'allyle. Après l'addition, l'agitation est ensuite poursuivie pendant 30 mn à température ambiante. La concentration du magnésien est alors de l'ordre de 0,84 mol/l.

Ethylènedioxy-1,1 hydroxy-3 méthyl-3 hexene-5 (B) :

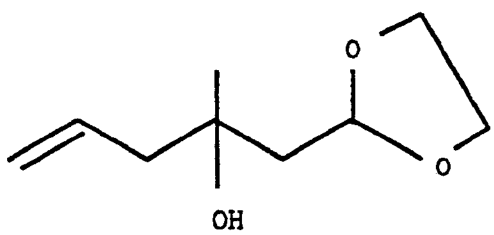

0,4 mol (51,6 g) d'éthylènedioxy-1,1 butanone-3 diluée dans 100 ml d'éther sont ajoutés lentement à la température de reflux de l'éther à la solution étherée de l'allylmagnésien. On agite pendant 45 minutes.

Le milieu réactionnel est refroidi à 0°C avant d'être hydrolysé à l'aide de 100 ml d'une solution aqueuse à 10% de chlorure d'ammonium. On observe alors la formation d'un précipité qui facilite le prélèvement de la phase éthérée. On reprend le solide dans un demi-litre d'eau et l'on extrait par trois fois 200 ml d'éther. Les phases éthérées sont rassemblées et concentrées. Le résidu est distillé. On obtient l'éthylène-1,1 hydroxy-3 méthyl-3 hexène-5.

Rdt : 80%. Eb : 110°C/18 mmHg.

IR : 3600-3200 (ν OH) ; 1640 (ν C=C)

Dibromo-5,6 éthylène dioxy-1,1 méthyl-3 hexanol-3 (C) :

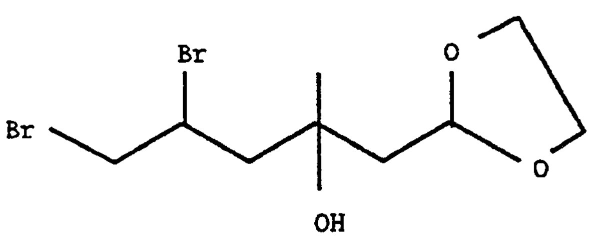

Dans un erlenmeyer à trois tubulures de 250 ml, équipé d'un septum, d'un thermomètre et d'une ampoule à brome, on introduit sucessivement 10 mmol d'hydroxyacétal dans 40 ml de tétrachlorure de carbone et 10

mmol de carbonate de sodium (1,06 g). Sous agitation, on ajoute en 5 min 10 mmol de brome (1,6 g) en solution dans 3 ml de solvant à une température de +3°C. On observe une décoloration immédiate de la solution bromée au contact du mélange réactionnel. Après l'introduction du brome, on agite 20 mn à cette température.

Après filtration, pour éliminer les produits insolubles la phase organique est lavée par 15 ml d'une solution aqueuse saturée d'hydrogénocarbonate de sodium. On extrait à l'éther, sèche sur sulfate de magnésium et on concentre. Le résidu est purifié par chromatographie éclair. On obtient le dibromo-5,6 éthylènedioxy-1,1 méthyl-3 hexanol-3 (éluant éther/éther de pétrole 25/100) avec un rendement de 80%.

Bromo-6 éthylènedioxy-1,1 hydroxy-3 méthyl-3 hexène-5 (D)

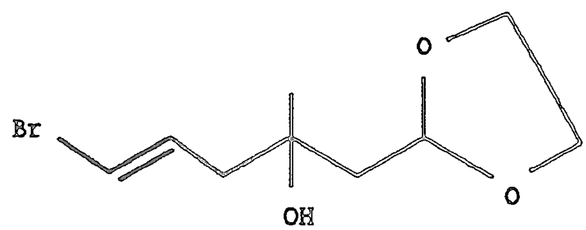

Le produit brut de la bromation de l'hydroxydioxolanne (C) est introduit dans un erlenmeyer sous atmosphère inerte, en solution dans 40 ml de THF anhydre. Après avoir baissé la température à -60°C, on ajoute 11 mmol de terbutanol (0,81 g) en solution dans 1 ml de THF. On additionne par petites fractions, en 20 minutes, 11 mmol de terbutylate de potassium (1,23 g). On laisse deux heures à cette température, la solution incolore du départ devient marron.

A cette même température, on ajoute rapidement 15 ml d'eau et le milieu réactionnel est agité fortement durant 30 minutes. On reprend à l'éther (50 ml) et la phase aqueuse est extraite à l'éther (7 fois 20 ml). On sèche sur sulfate de magnésium et concentre. Le résidu est alors purifié par chromatographie éclair. On isole l'hydroxy dioxolanne à brome vinylique (D).
Eluant : éther/éther de pétrole : 40/100.
Rdt : 56%. IR : 3600-3200 (ν OH) ; 1620 (ν C=C).

Bromation et déhydrohalogénation "in situ" sans isoler C

Dans un erlenmeyer à trois tubulures de 250 ml, équipé d'un septum, d'un thermomètre et d'une ampoule à brome, on introduit successivement 10 mmol d'hydroxyacétal (B) dans 40 ml de tétrachlorure de carbone. Sous agitation, on ajoute en 5 minutes, 11 mmol de brome (1,76 g) en solution dans 3 ml de solvant à une température de +3°C. On agite 20 minutes à cette température.

Le milieu réactionnel est ensuite dilué par 40 ml de tétrahydrofuranne anhydre et la température est portée à -60°C. 11 mmol de terbutanol (0,81 g) en solution dans 1 ml de THF sont additionnées puis 15 mmol (1,68 g) de terbutylate de potassium (toujours par petites fractions). On laisse remonter lentement à -20°C en une heure. La solution se colore en marron foncé.

L'hydrolyse est effectuée par 25 ml d'eau, à -20°C. On extrait à l'éther (7 fois 20 ml), sèche sur sulfate de magnésium et on concentre. Le résidu est purifié par chromatographie éclair. Dans ce cas, on isole 51% d'acétal à brome vinyligue (D).

II SYNTHESE DU RETINAL

Déshydratation des hydroxy acétals (B) et (D)

Dans un réacteur de 100 ml à trois tubulures, muni d'un thermomètre, d'une ampoule à brome et d'un réfrigérant, contenant une solution de 20 mmol d'hydroxyacétal dans 50 ml de dichlorométhane, on ajoute 69 mmol de triéthylamine (7 g), puis en 20 minutes, 46 mmol de chlorure de mésyle (5,3 g) dilué dans 5 ml de dichlorométhane. La réaction est exothermique et il y a apparition d'un précipité de chlorhydrate de triéthylamine. On laisse revenir à température ambiante (1 heure) avant de traiter par 50 ml d'eau distillée. On extrait par 5 fois 20 ml de dichlorométhane, sèche sur sulfate de magnésium et concentre la solution. Le produit brut est purifié par chromatographie éclair. On obtient :

à partir de (B) le dioxolanne du méthyl-3 hexadiène-3,5 al

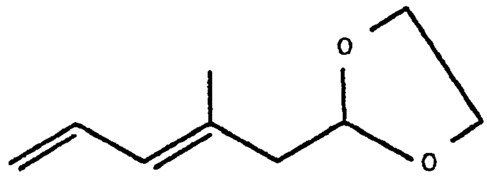

Eluant éther/éther de pétrole 10/100
Rdt = 64%. IR : 1640 (ν C=C)

à partir de (D) le dioxolanne du bromo-6 méthyl-3 hexadiène-3,5 al (E)

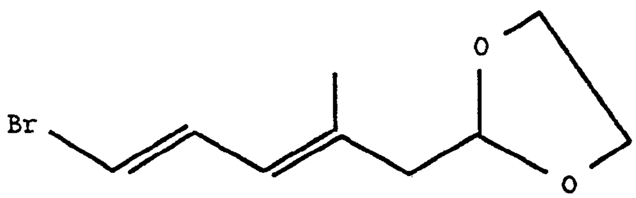

Eluant : éther/éther de pétrole : 10/100.
Rtd : 61%. IR : 1670-1660 (ν C=C).

Preparation de l'hydroxydioxolanne (F) à partir du dioxolanne du bromo-6 hexadiène-3,5 al (E)

Dans un bicol de 25 ml, on introduit sous argon 1,7 mmol du dioxolanne du bromo-6 méthyl-3 hexadiène-3,5 al (0,4 g) (E) en solution dans 10 ml d'éther anhydre. Le mélange est refroidi à -70°C avant d'ajouter en 10 minutes 3,4 mmol de tertiobutyllithium 1,6 N (2,12 ml). La température du milieu réactionnel est maintenue à -70°C pendant 90 minutes puis, on ajoute 1,5 mmol de β-ionone (0,29 g) en solution dans 2 ml d'éther anhydre.

Après 10 minutes à -70°C, on laisse remonter la température à -10°C en 15 minutes. On maintient cette température durant 90 minutes avant d'hydrolyser le milieu avec 3 ml d'une solution aqueuse saturée de bicarbonate de sodium. On extrait par 5 fois 15 ml d'éther, sèche sur sulfate de magnésium et concentre la solution. Le produit est purifié par chromatographie éclair. On obtient :

Dioxolanne du (triméthyl-2,6,6 cyclohexène-1)yl-9 diméthyl-3,7 hydroxy-7 nonatriène-3,5,8 (F)

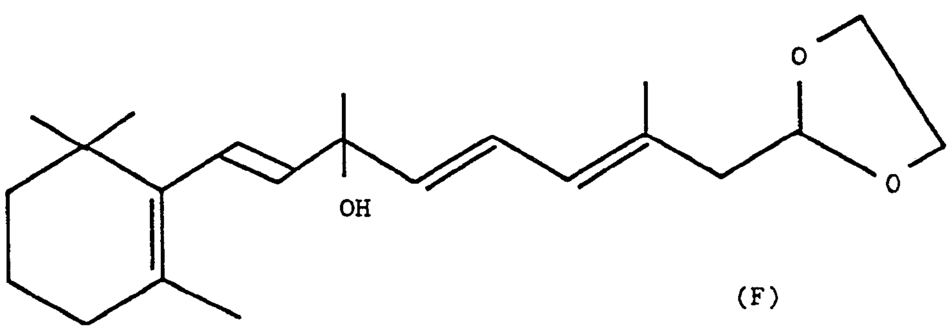

(F)

Eluant : éther/éther de pétrole : 30/100.
Rdt : 73%.
IR : 3600-3200 (ν OH) ; 1640 (ν C=C).

Hydrolyse de l'hydroxydioxolanne F

Dans un ballon tricol de 50 ml, on introduit successivement 1,1 mmol d'hydroxydioxolanne (0,38 g), 28 ml d'une solution de 192 ml d'acétone et de 1 ml d'eau, 0,02 g de ionol et 0,12 ml d'eau. On chauffe au reflux et l'on ajoute 0,2 ml d'une solution de 141 ml d'acétone et de 3 ml d'acide bromhydrigue aqueux à 48%. La réaction est suivie en chromatographie couche mince. On observe tout d'abord le produit de déhydratation (Rf : 0,8) et après 30 minutes de reflux le rétinal (Rf : 0,65).

Après avoir refroidi le milieu réactionnel à 0°C, on traite par 5 ml d'une solution aqueuse saturée de bicarbonate de sodium. On extrait par 5 fois 10 ml de pentane, sèche sur sulfate de magnésium et concentre. Le produit brut est purifié par chromatographie éclair. On obtient le rétinal.

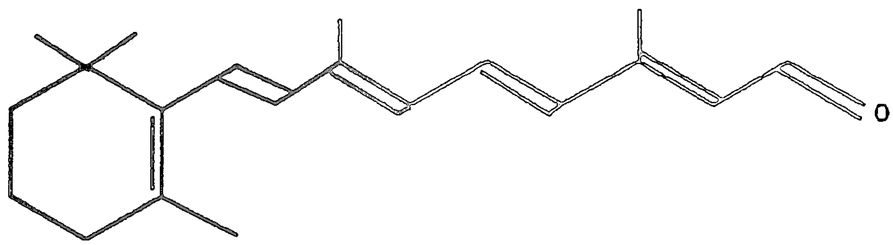

Eluant : éther/éther de pétrole : 10/100.
Rdt : 59%.
IR : 1670 (ν C=C) ; 1580 (ν C=C).
La RMN du proton indique la présence de deux signaux aldéhydiques dans un rapport de 70/30.

III SYNTHESE D'UN COMPOSE DE FORMULE (II)

Hydrolyse de l'hydroxy acétal (C),

Dans un erlenmeyer de 250 ml, équipé d'un thermomètre, d'un réfrigérant et d'une ampoule à brome, on introduit le produit brut (ou purifié) de la réaction de bromation de l'hydroxyacétal employé dans 100 ml d'acétone sec. On ajoute ensuite 1,4 ml d'eau puis 0,2 g de ionol. Le milieu réactionnel est chauffé au reflux pendant une minute avant l'introduction de 1,2 ml d'une solution composée de 142 ml d'acétone sec et de 3 ml d'acide bromhydrique aqueux à 48%. Le reflux est maintenu durant 40 minutes. La solution noircit à partir de la 35ème minute. Après disparition du spot de l'hydroxyacétal en CCM, on abaisse la température à +20°C, avant d'ajouter 100 ml de pentane. On traite par 10 ml d'une solution aqueuse saturée de bicarbonate de sodium pour ramener le milieu à un pH neutre. On extrait par 5 fois 30 ml de pentane, sèche au sulfate de magnésium et on concentre la solution. Le produit est purifié par chromatographie éclair. On obtient :

Bromo-6 méthyl-3 hexadiène-2,4 al (G)

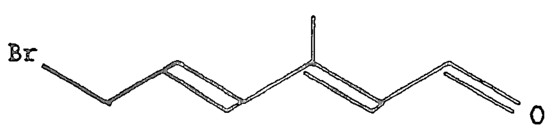

Eluant : éther/éther de pétrole : 10/100.
Rdt : 70%. IR : 1670 (ν C=O) ; 1650 (ν C=C).

Protection de la fonction aldéhyde par un groupement acétal :

A 5,85 mmol d'aldéhyde bromé (G) (1,13 g), on ajoute successivement 17,5 mmol d'orthoformiate mixte préparé par mélange de 0,2 mol d'orthoformiate de méthyle (21,2 g), 0,15 mol d'éthylène glycol (9,3 g) et d'une quantité catalytique d'acide paratoluène sulfonique porté au reflux et distillé. On introduit, à une température ambiante, 0,2 ml d'une solution de chlorure d'hydrogène à 12% dans le méthanol sec. Le degré d'avancement de la réaction est contrôlé en CCM. Après deux heures, le milieu réactionnel est neutralisé avec du méthylate de sodium. On évapore les solvants et l'on purifie le résidu par chromatographie éclair. On obtient :

Bromo-6 diméthoxy-1,1 méthyl-3 hexadiène-2,4 (H)

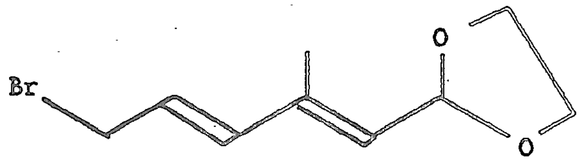

Eluant : éther/éther de pétrole : 5/100.
Rdt : 68%. IR : 1650 (ν C=C).

IV Preparation d'un composé de formule (III), le dioxolanne du diéthylphosphono-6 méthyl-3 hexadiène-2,4 al (I)

Dans un bicol de 50 ml, muni d'un thermomètre et d'un réfrigérant, on introduit successivement 2,56 g (15,44 mmol) de triéthyle phosphite dans 10 ml de toluène sec et 3 g (12,87 mmol) de bromo dioxolanne (H) dans 10 ml de toluène.

Le milieu réactionnel est chauffé au reflux du toluène durant 15 heures. Après disparition totale en CCM du spot de l'acétal bromé et élimination du solvant, le phosphonate acétal (I) est purifié par distillation sous pression réduite. On obtient :

Dioxolanne du diéthylphosphono-6 méthyl-3 hexadiène-2,4 al (I)

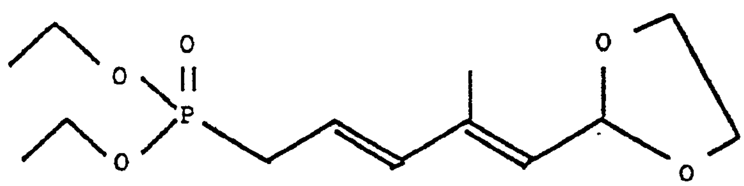

Eb : 165°C/0,3 mm Hg.
Rdt : 86%. IR : 1640-1620 (ν C=C).

Synthèse du rétinal

Sous argon, dans un bicol de 25 ml, on introduit 1,72 mmol de phosphonate (I) (0,5 g) en solution dans 10 ml de THF. On ajoute par petites fractions à -70°C, 2,22 mmol de tertiobutylate de potassium (0,275 g). On conserve l'agitation 90 minutes à -70°C. On ajoute ensuite 1,8 mmol de β-ionone (0,34 g) en solution dans un ml de THF. La température est maintenue 15 minutes à -70°C puis, 1 heure à -20°C.

L'hydrolyse du milieu réactionnel peut être effectuée selon deux procédé suivant que l'on souhaite isoler le rétinal ou sa forme protégée.

- Hydrolyse pour obtenir le rétinal :

On ajoute à -20°C une solution d'acide chlorhydrigue 3N (5 ml). Après un quart d'heure d'agitation, on extrait la phase aqueuse à l'éther (5 fois 15 ml), sèche sur sulfate de magnésium et concentre.

Le produit brut est purifié par chromatographie éclair. On obtient le rétinal.

Eluant : éther/éther de pétrole : 10/100.

Rdt : 62%.

IR : 1670 (ν C=O) ; 1580 (ν C=C).

La RMN du proton indique la présence de deux signaux aldéhydiques dans un rapport de 73/27.

- Hydrolyse pour obtenir la forme protégée.

On verse le milieu réactionnel dans 10 ml d'eau glacée. Les traitements suivants sont les mêmes que précédemment

Dioxolanne du rétinal

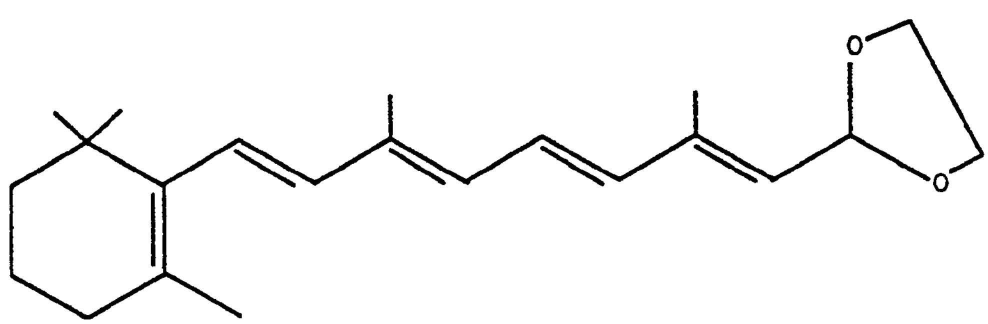

Eluant : éther/éther de pétrole 4/100
Rdt : 60%
IR : 1660 (ν C=C)
Autres poduits préparés selon le mode opératoire précédent :

A partir de l'acétone : Dioxolanne du déhydrocitral

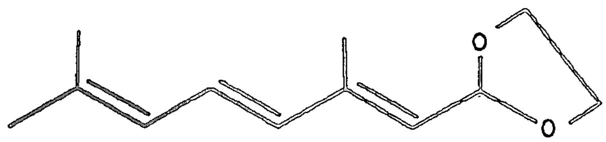

Eluant : éther/éther de pétrole
Rdt : 53%
IR : 1650-1630-1600 (ν C=C)

A partir de la méthylhepténone : Dioxolanne du déhydrofarnésal

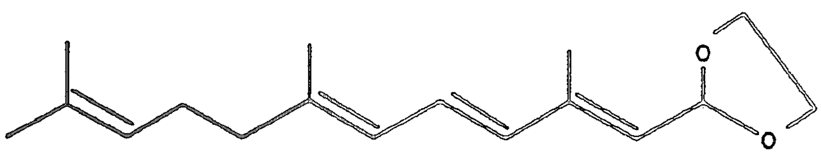

Eluant : éther/éther de pétrole
Rdt : 45%
IR : 1660-1650 (ν C=C)

**Revendications**

1. Nouveaux dérivés intermédiaires répondant à la formule :

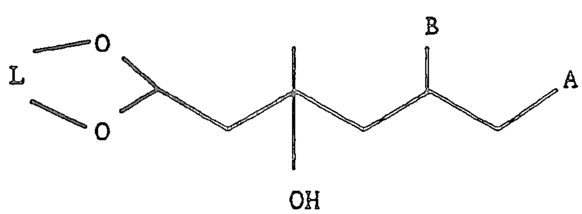

(I)

dans laquelle
- L représente un radical alkylène contenant 2 à 3 atomes de carbone éventuellement substitué par un ou plusieurs groupes alkyle, alkylène ou alkoxy contenant 1 à 4 atomes de carbone,
- A représente un halogène choisi parmi le chlore, le brome, l'iode et/ou une liaison covalente avec B ;
- B représente un halogène choisi parmi le chlore, le brome, l'iode ou une liaison covalente avec A,

2. Nouveaux intermédiaires selon la revendication 1 caractérisés en ce que A représente une liaison covalente avec B.

3. Nouveaux intermédiaires selon la revendication 1 caractérisés en ce que A représente un halogène, B une liaison covalente avec A.

4. Nouveaux intermédiaires selon la revendication 1 caractérisés en ce que A et B représentent un halogène.

5. Procédé de préparation des intermédiaires selon la revendication 2 caractérisé en ce que dans une première étape on met en présence l'acétal dialkylique de l'acétylacétaldéhyde avec un dialcool en présence

d'un catalyseur acide puis dans une deuxième étape le dérivé obtenu à la première étape avec un halogénure d'allyl magnésium.

6. Procédé selon la revendication 5 caractérisé en ce que l'acétal dialkyligue de l'acétylacétaldéhyde est un acétal dont la chaîne alkyle contient 1 à 4 atomes de carbone et de préférence l'acétal diméthyligue de l'acétylacétaldéhyde.

7. Procédé selon la revendication 5 caractérisé en ce que le dialcool est un dialcool aliphatique contenant 2 à 3 atomes de carbone éventuellement substitué par un ou plusieurs groupes alkyle, alkylène ou alkoxy contenant 1 à 4 atomes de carbone.

8. Procédé de préparation selon la revendication 5 caractérisé en ce que le dialcool est l'éthylène glycol ou le propylène glycol.

9. Procédé de préparation des intermédiaires selon la revendication 3 caractérisé en ce qu'on met en présence l'intermédiaire de la revendication 2 avec le brome.

10. Procédé de préparation des intermédiaires selon la revendication 4 caractérisé en ce que l'on met en présence l'intermédiaire de la revendication 2 avec le brome en présence d'une base faible. Procédé de préparation selon l'une quelconque des revendication 9 ou 10 caractérisé en ce que le solvant utilisé est un solvant halogéné ou un hydrocarbure.

11. Nouveaux intermédiaires répondant à la formule (II)

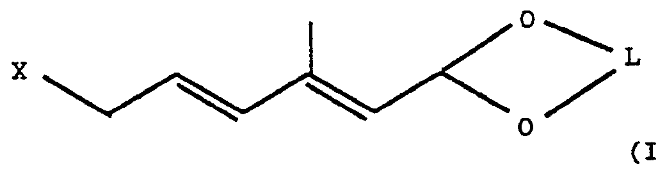

(II)

dans laquelle L a la même signification que dans la revendication 1, X représente la chlore, le brome ou l'iode

12. Procédé de préparation des intermédiaires selon la revendication 12 caractérisé en ce que dans une première étape on met en présence l'intermédiaire de la revendication 4 avec un acide fort et dans une deuxième étape on met en présence le dérivé issu de la première étape avec un dialcool selon l'une quelconque des revendications 7 ou 8 ou un orthoformiate mixte.

13. Nouveaux intermédiaires répondant à la formule (III) :

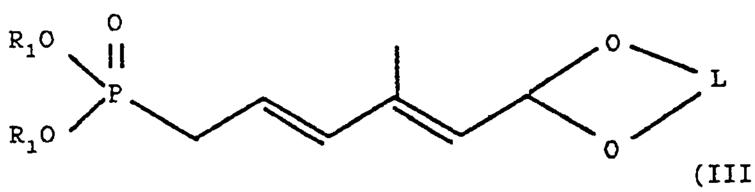

(III)

dans laquelle $R_1$ représente un groupe alkyle linéaire ou ramifié contenant 1 à 6 atomes et L a la même signification que dans la revendication 1.

14. Procédé de préparation des intermédiaires selon la revendication 14 caractérisé en ce que l'on met en présence le dérivé de formule (II) et un trialkylphosphite dans un solvant organique.

15. Utilisation des composés de formule (III) pour la préparation du rétinal caractérisé en ce qu'on le met en présence avec la β-ionone en présence d'une base.

16. Utilisation des composés de formule (II) pour la préparation du rétinal caractérisé en ce que l'on met en présence avec une sulfone de formule :

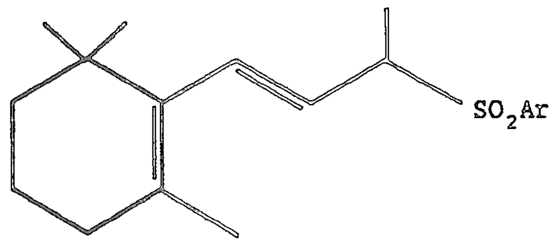

dans laquelle Ar représente un radical aromatique en présence d'un agent basique choisi parmi les hydroxydes, les alcoolates, les hydrures ou amidures alcalins.

17. Utilisation des composés selon la revendication 4 pour la préparation du rétinal caractérisé en ce que dans une première étape on effectue une deshydrohalogénation au moyen d'une base, puis dans une deuxième étape, on effectue une deshydratation au moyen d'un halogénure et d'une base, dans une troisième étape on effectue un échange halogène metal au moyen d'un alkyllithium, dans une quatrième étape on condense le dérivé obtenu à la troisième étape avec la β-ionone, dans une cinquième étape on élimine le groupe protecteur au moyen d'un acide fort dans un solvant organique.

18. Utilisation des composés selon la revendication 3 pour la préparation du rétinal, caractérisé en ce que l'on utilise les étapes 2 à 5 de la revendication 18.

Office européen des brevets

# RAPPORT DE RECHERCHE EUROPEENNE

Numero de la demande

EP 90 40 3395

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | DE-A-2 708 281 (RHONE-POULENC)<br>* Revendication 1; pages 5-6 *<br>--- | 12 | C 07 D 317/20<br>C 07 D 317/16<br>C 07 F 9/655<br>C 07 D 403/14<br>C 07 C 317/10 |
| X | DE-A-2 708 304 (RHONE-POULENC)<br>* Revendication 1; pages 5-6 *<br>--- | 12 | |
| A | JOURNAL OF ORGANIC CHEMISTRY, vol. 50, no. 12, 14 juin 1985, pages 2011-2019, American Chemical Society, Washington, DC, US; G.C. FISCHER et al.: "Irreducible analogues of mevaldic acid coenzyme A hemithioacetal as potential inhibitors of HMG-CoA reductase. Synthesis of a carbon-sulfur interchanged analogue of mevaldic acid pantetheine hemithioacetal"<br>* Page 2012, composé 10a *<br>--- | 1,2 | |
| A | EP-A-0 237 438 (RHONE-POULENC)<br>* En entier *<br>----- | 18,19 | |
| | | | **DOMAINES TECHNIQUES RECHERCHES (Int. Cl.5)** |
| | | | C 07 D 317/00<br>C 07 F 9/00<br>C 07 C 403/00 |

Le présent rapport a été établi pour toutes les revendications

| Lieu de la recherche | Date d'achèvement de la recherche | Examinateur |
|---|---|---|
| LA HAYE | 20-02-1991 | ENGLISH R.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)